(19) Europäisches Patentamt
European Patent Office
Office européen des brevets

(11) Publication number: 0 000 036
B1

(12) EUROPEAN PATENT SPECIFICATION

(45) Date of publication of patent specification: 14.10.81

(21) Application number: 78100059.1

(22) Date of filing: 01.06.78

(51) Int. Cl.³: C 07 C 87/28, C 07 C 91/30, C 07 C 91/32, C 07 C 93/26, C 07 C 101/10, C 07 C 101/24, C 07 D 233/64, C 07 D 209/16 //A61K31/135, A61K31/195, A61K31/40, A61K31/415

# (54) Fluorinated alkylamines and process for preparing same.

(30) Priority: 01.06.77 US 802350

(43) Date of publication of application:
20.12.78 Bulletin 78/1

(45) Publication of the grant of the European patent:
14.10.81 Bulletin 81/41

(84) Designated Contracting States:
BE CH DE FR GB LU NL SE

(56) References cited:
GB - A - 976 353
GB - A - 1 218 135
US - A - 3 839 170

(73) Proprietor: MERCK & CO. INC.
126, East Lincoln Avenue P.O. Box 2000
Rahway, New Jersey 07065 (US)

(72) Inventor: Kollonitsch, Janos
3 Plymouth Road
Westfield, N.J. (US)
Inventor: Patchett, Arthur Allan
207 Oak Lane
Cranford, N.J. (US)

(74) Representative: Abitz, Walter, Dr.-Ing. et al,
Abitz, Morf, Gritschneder P.O. Box 86 01 09
D-8000 München 86 (DE)

EP 0 000 036 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Fluorinated alkylamines and process for preparing same

## Background of the Invention

The present invention is concerned with novel 1-fluoromethyl substituted alkylamines.

Various nonfluorinated substituted alkylamines such as histamine, 2-(3,4-dihydroxyphenyl) ethylamine (dopamine), tyramine, amphetamine and hydroxyamphetamine, are known. These compounds exhibit various physiological activities and have various clinical utilities (See D. M. Aviado "Sympathomimetic Drugs", Charles C. Thomas, Publisher, 1970). G.B. 976,353 discloses 1-trifluoromethylphenylalkylamines having anorectic properties. No 1-monofluoromethylaralkylamines are suggested. U.S. 3,839,170 discloses $\alpha$-$CH_2F$ amines and amino acids and the process for direct replacement of a hydrogen atom with a F atom using fluoroxyperfluoroalkanes and fluoroxypentafluorosulfur in liquid or solid phase under specific reaction conditions. No 1-fluoromethylamines of the type disclosed in the present invention are suggested.

1-Fluoromethyl substituted alkyl amines have been discovered. These amines have decarboxylase inhibiting activity.

## Summary of the Invention

1-Fluoromethyl substituted alkylamines and salts thereof.

## Description of the Invention

An embodiment of the present invention is compounds having the formula

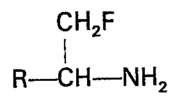

I

wherein R is a substituted $C_1$—$C_4$ alkyl group.

The pharmaceutically acceptable acid addition salts of the formula I compounds are also included. In general, the salts are those of the formula I base with a suitable organic or inorganic acid. Preferred inorganic acid salts are the hydrohalides e.g., hydrochlorides, hydroiodides, hydrobromides; the sulfates, and the phosphates. The hydrohalides, and especially the hydrochlorides, are more preferred.

The formula I compounds have a chiral center and may occur in optically active forms i.e., as optical isomers. These isomers are designated conventionally by the symbols L and D, + and —, l and d, S and R or combinations thereof. Where the compound name or formula has no isomer designation, the name or formula includes the individual isomers, mixtures thereof and racemates.

The compounds having the S-isomer configuration are, in general preferred.

R is a substituted $C_1$—$C_4$ alkyl group exemplified by

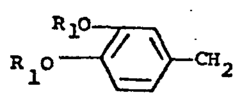

where $R_1$ is H or $C_2$—$C_6$ alkanoyl e.g., $CH_3$—CO, $CH_3$ $(CH_2)_4$—CO, $(CH_3)_3C$—CO and the like;

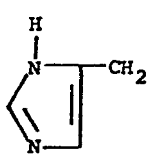 , 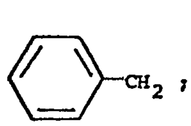 ; 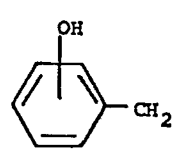 preferably

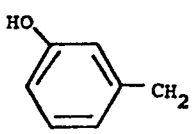 and 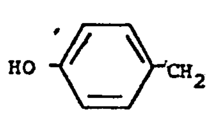 ; 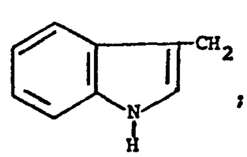 ;

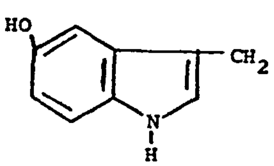 ;

$HOOC—CH_2—CH_2$; 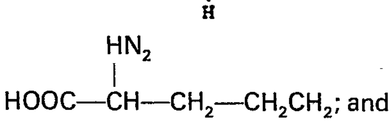 ; and 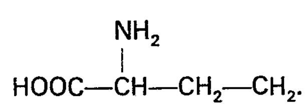 .

Preferred compounds of formula I are those where R is

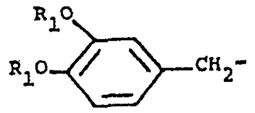 , 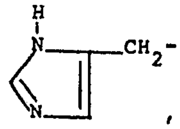 , 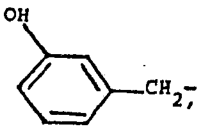

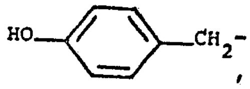 , 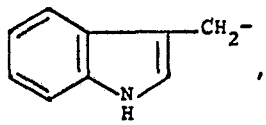 ,

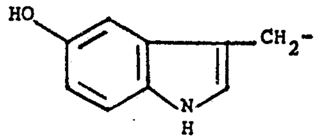 or $HOOC—CH_2—CH_2—$.

Compounds which are particularly preferred have the formula

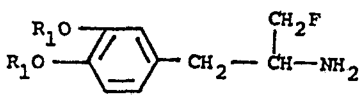

II

More preferred formula II compounds are those wherein $R_1$ is hydrogen; formula II compounds having the S-isomer configuration are especially preferred.

Another particularly preferred compound has the formula

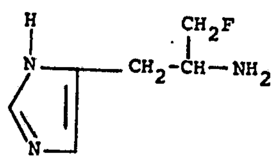

III

Another preferred compound has the formula

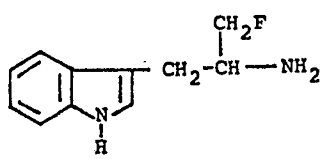

IV

Another preferred compound has the formula

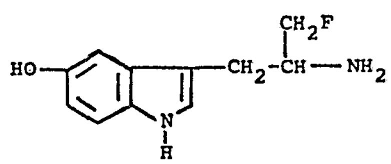

V

Still another preferred compound has the formula

$$\begin{array}{c} \qquad\qquad\qquad\qquad\quad CH_2F \\ \qquad\qquad\qquad\qquad\quad | \\ HOOC—CH_2—CH_2—CH—NH_2 \end{array}$$

VI

The compounds of the present invention have potent decarboxylase inhibiting activity. Decarboxylases are enzymes which act on $\alpha$-amino acid substrates, effecting decarboxylation to produce the corresponding amine. This action is illustrated by the following equation:

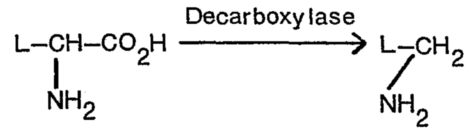


L = alkyl or aralkyl group

α-amino acid substrate amine

By inhibiting this decarboxylation, the biosynthetic pathway to a number of biologically significant amines can be modulated or inhibited with physiologically useful consequences. For example, α-fluoromethyl dopamine inhibits dopa decarboxylase and can be used in combination with dopa to potentiate the latter's usefulness in the treatment of Parkinson's disease.

The present compounds also are substantially specific in their decarboxylase inhibition activity, that is an α-fluoromethyl substituted alkylamine generally inhibits the decarboxylation of the corresponding non α-fluoromethyl-α amino acid. For example, α-fluoromethyl dopamine inhibits the decarboxylation of dopa; α-fluoromethyl histamine will inhibit the decarboxylation of histidine; 4-FM-GABA (4-fluoromethyl-4-amino-butyric acid) inhibits glutamic acid decarboxylase; etc.

Because of this specificity and potency as decarboxylase inhibitors, the present compounds are also useful as diagnostic tools to determine the presence and importance of the corresponding decarboxylase in relation to diseases or to the functioning of biological systems. For example, the role of catechol amines in certain CNS (central nervous system) functions can be studied by inhibiting their biosynthesis with an appropriate α-fluoromethyl-alkylamine; α-fluoromethyl-tryptamine displays antihypertensive activity; and the study and treatment of ulcers can be advanced through modulation of histamine biosynthesis using α-fluoromethyl histamine.

Representative compounds have been determined to have decarboxylase inhibiting activity using a conventional in-vitro assay.

A representative compound, S—(and R) 1 - fluoromethyl - 2(3,4 - dihydroxyphenyl)-ethylamine, also referred to as α-fluoromethyl dopamine, was found to effect a pressor response in rats. This indicates that some of the present compounds may also be useful for treating hypotension in humans.

4-FM-GABA displays CNS activities, including sedative and antidepressant indications.

The compounds of the present invention may be prepared using any convenient method.

One such useful process involves the reaction of an α-hydroxymethyl substituted alkyl amine with $SF_4$ in liquid HF, as illustrated by the following equation:

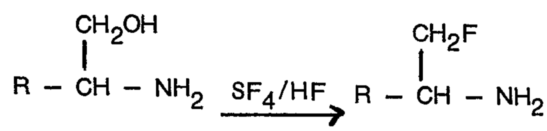


The starting compounds as

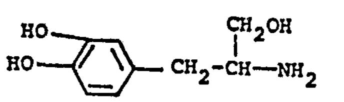 , 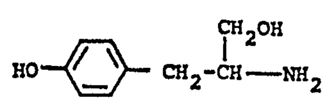 ,

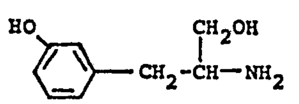 , 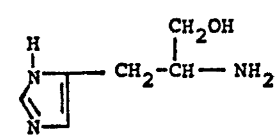 ,

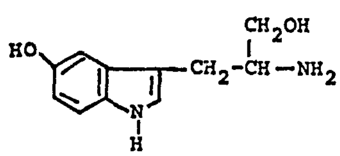 , 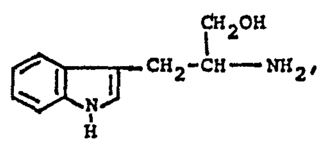 ,

or 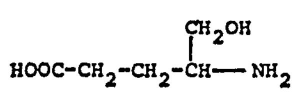

can be obtained according to ENz et al., Helv. Chim. Acta 29, 1048—1060 (1946). The reaction is generally carried out at temperatures ranging from about −80°C to about 20°C. This general reaction is also referred to as fluorodehydroxylation and is described in the Journal of Organic Chemistry *40*, 3809—10 (1975).

Another useful method for preparation of α-fluoromethyl-substituted amines involves photofluorination, e.g.:

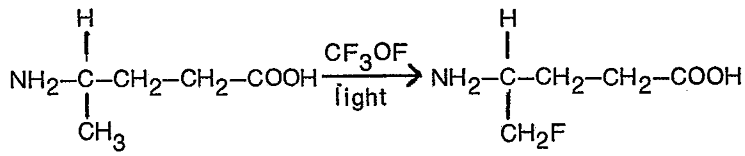


This process was described generally in J. Am. Chem. Soc. *98*, 5591 (1976) and ibid. *92*, 7494 (1970).

An acid addition salt of a compound of the present invention may be prepared by conventional treatment of the α-fluoromethyl-substituted amine with a useful acid generally in a suitable solvent.

A single enantiomer of the present compounds may be obtained by (1) resolving the fluoromethyl-substituted amine racemate using conventional resolution techniques or (2) resolving the precursor α-hydroxymethyl-substituted amine using conventional resolution techniques and then fluoro-dehydroxylating the precursor enantiomer. A conventional resolution technique may involve formation of a salt of the appropriately substituted amine with an optically active acid and subsequently recovering the specific enantiomer from the salt.

Compounds of the formula

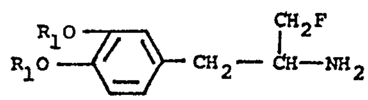

where $R_1$ is $C_2$—$C_6$ alkanoyl are prepared by acylating the corresponding compound where $R_1$ is hydrogen, in an acid medium to prevent acylation of the amino group. In general, conventional acylating agents and conditions are employed.

The following examples illustrate preparation of representative compounds of the present invention. All temperatures are in °C. Melting points are determined in open capillary and are uncorrected.

Example 1

*Synthesis of R-α-Fluoromethyl-Dopamine-HCl*

*a) Preparation of R-α-Hydroxymethyl-Dopamine·HCl*

Four and 55/100 g of sodium borohydride is suspended by stirring (magnetic stirring bar) with 250 ml of tetrahydrofuran (THF). To the stirred suspension there is added 6.7 g of $CaCl_2$ (powder), the mixture stirred for 30 minutes at room temperature, then refluxed under stirring for 90 minutes. To the $Ca(BH_4)_2$ solution thus obtained, a solution of 10.2 g of methyl ester of *D*-DOPA (DOPA = 3,4-dihydroxy-phenylalanine) in 55 ml of THF is admixed. After 15 minutes at room temperature, the suspension is refluxed with continued stirring for 5-1/2 hours. (Note: The whole operation described above was conducted under a protective blanket of dry $N_2$ gas.) The solvent is evaporated in vacuo and methanol (300 ml) is added with caution. After the gas evolution ceases, the solvent is removed by distillation in vacuo, fresh methanol is added again, the HCl gas is passed in until saturation. Solvent is removed again by evaporation in vacuo and the whole $CH_3OH$/HCl treatment is repeated. (These treatments split the borate complex formed in the reduction as well as removing the methyl borate). The residue is dissolved in $H_2O$, 109 ml of 0.55M aq. $H_2SO_4$ is added and the mixture aged at 5°C overnight. The $CaSO_4{\cdot}2H_2O$ is then removed by filtration, washed with water (10 ml) and isopropanol (2 × 30 ml). The combined filtrates were evaporated in vacuo to dryness, the solid residue is stirred with isopropanol (100 ml) for 1/2 h., filtered, cake washed with isopropanol (2 × 30 ml), the combined filtrates evaporated to dryness in vacuo. This crude product is further purified by elution chromatography (cation-exchange resin column made of 0.95 1 AG50—X—8 resin, 200/400 mesh U.S. Bureau of Standards, Standard Screen Sieves 1919; H form). Effluent is monitored by LKB UVICORD II UV monitor. Elution is as follows:

0.4M aq. HCl, containing 7.5% methanol: 5 l
0.6M aq. HCl, containing 10% methanol: 4.8 l
0.7M aq. HCl, containing 10% methanol: 2.1 l
1.0M aq. HCl, containing 10% methanol: 4.0 l

Elution rate: 0.6 1/hour.

The product is located by the UV absorption monitor, which is connected with a chart recorder. The UV absorbing peak is released by the last solvent listed. The appropriate fractions are combined and evaporated to dryness in vacuo, to deliver the hydrochloride of *R* - α - hydroxymethyl -

dopamine. For final purification this is recrystallized from isopropanol, to give crystalline product, m.p. 159—160°C. $[\alpha]_D$: 19.5 ± 0.5° (c, 1 in 1M aq. HCl).

b) *Preparation of R-α-Fluoromethyl-Dopamine·HCl*

One g of the product obtained under a) is charged into a KEL-F (Registered Trade Mark) reactor. HF gas is passed in while the reactor is immersed in a dry-ice-acetone bath, until a solution with 30 ml volume forms. The cooling bath is removed and the solvent evaporated by passing through a stream of $N_2$ gas. The residue thus obtained represents the HF salt of *R*-α-hydroxymethyl-dopamine. This is redissolved by condensing into the reactor HF again by cooling it in a dry-ice-acetone bath and passing in HF gas until a solution with volume 50 ml forms. $SF_4$ gas (1.5 ml, measured as liquid at −78°C) is passed in then under continuous cooling and stirring and the solution left standing overnight, while the reactor is being kept in the cooling bath, but without replenishing dry ice. The solvent is removed the next morning by passing through a stream of $N_2$ and the residue is redissolved in 2.5 M aq. HCl (25 ml), evaporated to dryness and the residue purified by elution chromatography on a column made of cation-exchange resin (190 ml of Dowex 50 AG50-X-8, (Registered Trade Mark) 200/400 mesh U.S. Bureau of Standards, Standard Screen Sieves 1919). Elution with water, followed by 0.5M aq. HCl with 5% methanol (2 l), followed by 0.6M aq. HCl with 10% methanol (4 l). UV absorption of the effluent is followed by LKB UVICORD II recording UV monitor. The effluent fractions containing UV absorbing material are evaporated to dryness in vacuo to deliver *R*-α-fluoromethyl-dopamine·HCl. 0.6 g of this crude product is dissolved in isopropanol (4 ml), treated with DARCO G-60 (Registered Trade Mark) then 26 ml of ethyl acetate is added. The crystalline product is recrystallized once more in a similar manner, to give 0.545 g of pure HCl salt, m.p. 152—3°C $[\alpha]_D$: 18.4 ± 0.5 (C, 1 in 1M aq. HCl).

Example 2

*Synthesis of S-α-Fluoromethyl-Dopamine·HCl*

S-α-Fluoromethyl dopamine·HCl is synthesized in an entirely analogous manner as described in example 1 for the *R* isomer; however to obtain the *S* isomer, the methyl ester of *L-DOPA* is employed as starting material. The intermediate *S*-hydroxymethyl dopamine has a melting point of 159—60°C; $[\alpha]_D$: −20.1 ± 0.5° (C, 1 in 1M aq. HCl). The *S*-α-fluoromethyl-dopamine·HCl obtained from this fluorodehydroxylation, has a m.p. = 151—3°C, $[\alpha]_D$: −19.2 ± 0.5° (C, 1 in 1M aq. HCl).

Example 3

*Synthesis of R-α-Fluoromethyl-Histamine*

One g of *D*-histidinol is placed into a KEL-F reactor; the reactor is immersed into a dry-ice-acetone cooling bath and HF gas is passed in until a volume of 40 ml collects. $SF_4$ gas is passed in (2.0 ml, measured as liquid, at −78°C) and the mixture kept at −78°C for 5 hours. The cooling bath is removed and the solvent evaporated by passing $N_2$ gas through it. The residue is dissolved in cc. aq. HCl (15 ml), the solution is evaporated to dryness in vacuo to yield substantially pure *R*-α-fluoromethyl-histamine hydrochloride-hydrofluoride salt. For transformation into the dihydrochloride salt, this product is dissolved in water and charged onto a cation-exchange resin column Dowex 50-X-8 (resin, $H^+$ form). The column is washed first with $H_2O$ until the effluent becomes neutral, then the product is eluted with 4M aq. HCl (275 ml). This effluent is evaporated to dryness to deliver substantially pure *R*-α-fluoromethyl-histamine dihydrochloride. This is recrystallized by dissolving it in 40 ml of boiling ethanol 2BA, concentrating this solution by evaporation in vacuo to 15 ml volume and cooled (ice-bath) for 2 hours. The crystals formed are collected by filtration and dried in vacuo to give *R*-α-fluoromethyl-histamine dihydrochloride, m.p. 181—2°.

Example 4

*Synthesis of S-α-fluoromethyl-Histamine*

*S*-α-Fluoromethyl histamine·HCl is prepared from L-histidinol via the method described in Example 3 for *R*-α-fluoromethyl-histamine·2HCl, m.p. = 182—83°C.

The free amines are obtained from the hydrochloride salts by conventional neutralization.

Example 5

*Synthesis of (S)-α-fluoromethyl-tryptamine*

S(L)-Tryptophanol (0.7 g, 3.7 mmoles) was placed in a Kel-F reactor, cooled in a dry ice-acetone bath (−78°C) and approximately 20 ml of anhydrous HF was condensed with stirring at −78°C. Sulfur tetrafluoride (approx. 1.5 ml, 26 mmoles) was added with stirring at −78°C over a 15-minute period. The reaction mixture was stirred for 30 minutes at −78°C and then the HF was blown off with a fast stream of $N_2$ over a 2.5-hr. period at −78°C. The dark residue was dissolved in 25 ml 3N HCl and evaporated to dryness at 25°C, in vacuo. The residue was dissolved in 10 ml $H_2O$, basified with 2.5N NaOH, and the separated amine was extracted with 2 x 50 ml ether. The combined ether extracts were dried over $MgSO_4$ and evaporated to dryness in vacuo at room temperature. The crude product (4 spots, by TLC on silica gel plates developed with ethyl acetate-methanol-water 85:10:5) was chromatographed on silica gel H (E. Merck, 120g.) using ethyl acetate-methanol-water 88:10:2 as the

elution solvent. The desired product (S)-α-fluoromethyl-trypamine, was contained in fractions No. 31—60 (12 ml each). They were combined and evaporated to dryness to yield S-α-fluoromethyl-tryptamine characterized as the tartrate salt, by 300 MHz $^1H$ NMR, mass spectroscopy and microanalysis.

Example 6

*Synthesis of R-α-Fluoromethyl-tryptamine*

Employing the procedure described in Example 5, but using R(D)-tryptophanol as starting material, R-α-fluoromethyl-tryptamine is obtained.

Example 7

*Synthesis of R,S-4-Fluoromethyl-4-Amino-butyric Acid*

Eleven and 7/10 g of 4-methyl-4-aminobutyric acid is placed in a KEL-F reactor and dissolved in 200 ml of liquid HF; then $CF_3OF$ gas is passed in while the reactor is immersed in a dry-ice/acetone cooling bath. The solution is irradiated (through a window on the top) by a 2500 W ultraviolet light source. (See J. of Am. Chem. Soc. *98*, 5591-93 (1976) and ibid *92*, 7494 (1970) for a general description of photofluorination).

First, 3 ml liquid $C_3OF$ is allowed to evaporate and passed into the solution during a 70 minute period, followed by another 40 min. period with irradiation. Two additional 2 ml (liq.) increments of $CF_3OF$ are passed into the reaction mixture with continuing irradiation, each in a time period of about 2 hours. The liquid HF is then removed by a stream of nitrogen gas. The residue is dissolved in 50 ml of 2.5 N aq. HCl and evaporated to dryness in vacuo. This treatment is repeated. The residue thus obtained is dissolved in 270 ml of water; 270 ml of conc. aq. HCl is added and the solution refluxed for 16 hours, then evaporated to dryness in vacuo, redissolved in 100 ml of water and chromatographed on a column of cation-exchange resin. 3 l of AG-50-X-8 (200—400 mesh, U.S. Bureau of Standards, Standard Screen Sieves 1919) resin is employed in the $H^+$ form. Elution: 18 liters of water, followed by 0.4 N aq. HCl. The effluent is monitored by UVICORD Model III ultra-violet absorption monitor, filter 206 nm. 22 ml fractions are collected. Fractions 410—610 are combined and evaporated to dryness in vacuo to deliver 4 - fluoromethyl - 4 - amino-butyric acid hydrochloride. For liberation of the acid, the 4—FM—GABA·HCl is dissolved in water and passed through an AG-50-X-8 ion-exchange resin column (100 ml of resin). The column is first washed with water, then eluted with 2 N aq. $NH_4OH$. Evaporation of the $NH_4OH$ solution in vacuo gives *R,S* - 4 - fluoromethyl - 4 - amino butyric acid. It is recrystallized from $H_2O$/isopropanol and characterized by C—H—N—F analysis and $^1H$ and $^{19}F$ NMR spectroscopy.

**Claims**

1. A compound of the formula

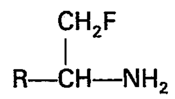

wherein R is a substituted $C_1$—$C_4$ alkyl group.

2. Pharmaceutically acceptable acid addition salts of the Claim 1 compounds.

3. A compound of Claim 1 having the S-isomer configuration.

4. A compound of Claim 1 wherein said substituted alkyl group is

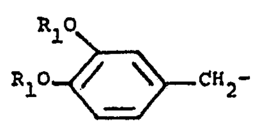

wherein $R_1$ is H or $C_2$—$C_6$ alkanoyl

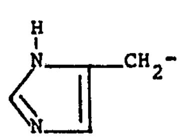

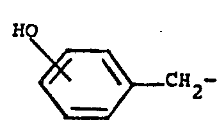

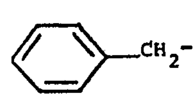

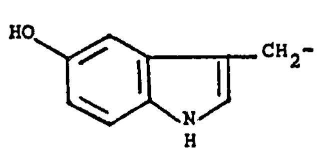

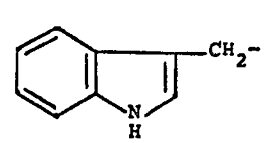

$$HOOC—CH_2—CH_2; \quad HOOC—\overset{NH_2}{\underset{H}{C}}—CH_2—CH_2; \text{ or } HOOC—\overset{NH_2}{CH}—CH_2CH_2—CH_2$$

5. A compound of Claims 3 or 4 wherein said substituted alkyl group is

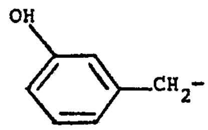 or 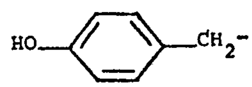

6. A compound of claim 3 wherein said substituted alkyl group is

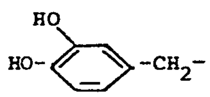 ; 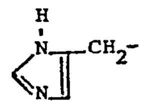 ; 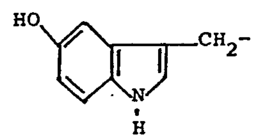 ;

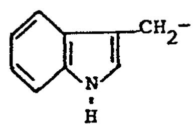 or HOOC—$CH_2$—$CH_2$—

7. A process for preparing compounds of Claim 1 which comprises fluorodehydroxylation of a compound having the formula

$$R—\overset{CH_2OH}{CH}—NH_2 \qquad VII$$

wherein R is a substituted $C_1$—$C_4$ alkyl group.

8. The process of Claim 7 wherein said formula VII compound is

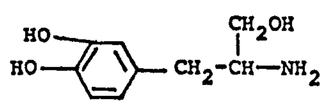 , 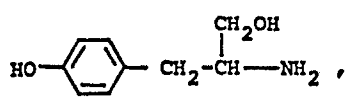 ,

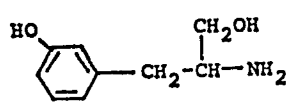 , 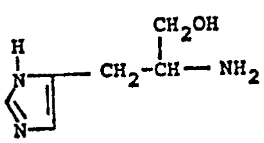 ,

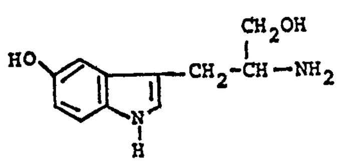 , 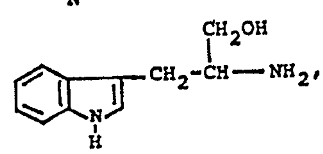 ,

or 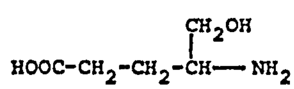

**Revendications**

1. Composé de formule

$$R—\overset{CH_2F}{CH}—NH_2$$

où R est un groupe alcoyle en $C_1$ à $C_4$ substitué.

2. Sels d'addition acides pharmaceutiquement acceptables des composés de la revendication 1.

3. Composé de la revendication 1 ayant la configuration isomérique S.

4. Composé de la revendication 1 où ledit groupe alcoyle substitué est

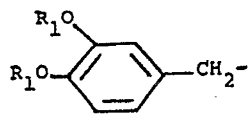

où $R_1$ représente H ou un alcanoyle en $C_2$ à $C_6$,

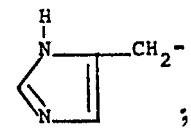 ; 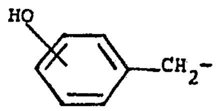 ; 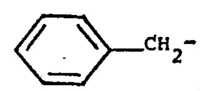 ;

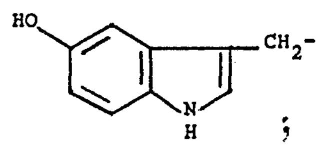 ; 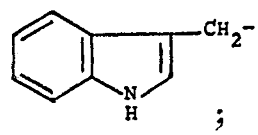 ;

$$HOOC—CH_2—CH_2, \quad HOOC—\overset{HN_2}{\underset{H}{C}}—CH_2—CH_2; \text{ ou } HOOC—\overset{NH_2}{CH}—CH_2CH_2—CH_2—$$

5. Composé des revendications 3 ou 4 où ledit groupe alcoyle substitué est

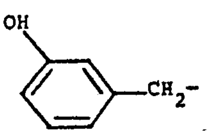 ou 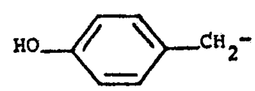

6. Composé de la revendication 3 où ledit groupe alcoyle substitué est

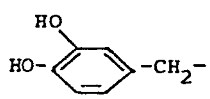 ; 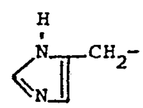 ; 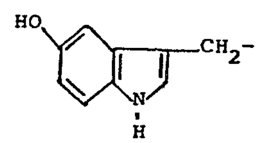 ;

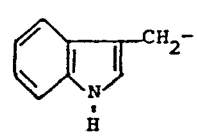 ou $HOOC—CH_2—CH_2—$.

7. Procédé de préparation des composés de la revendication 1 qui comprend la fluorodéshydroxylation d'un composé de formule

$$R—\overset{CH_2OH}{CH}—NH_2 \qquad VII$$

où R est un groupe alcoyle en $C_1$ à $C_4$ substitué.

8. Procédé de la revendication 7 où ledit composé de formule VII est

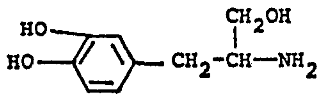 ,
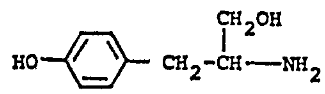 ,

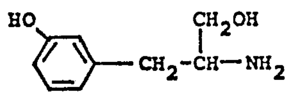 ,
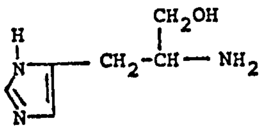 ,

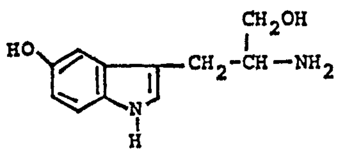 ,
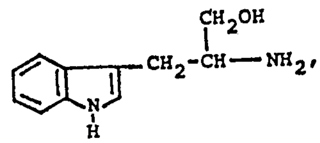 ,

ou 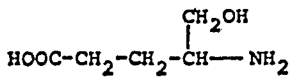

**Patentansprüche**

1. Verbindung mit der Formel

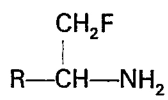

worin R eine substituierte $C_1$—$C_4$-Alkylgruppe ist.

2. Pharmazeutisch brauchbare Säureadditionssalze der Verbindungen des Anspruchs 1.

3. Verbindung nach Anspruch 1 mit der Konfiguration des S-Isomeren.

4. Verbindung nach Anspruch 1, in der die substituierte Alkylgruppe

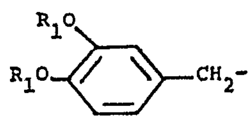

worin $R_1$ H oder $C_2$—$C_6$-Alkanoyl ist,

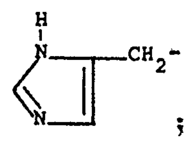 ; 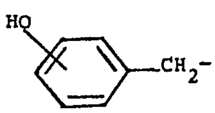 ; 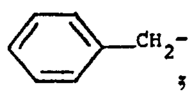 ;

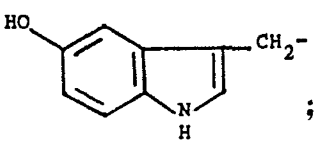 ; 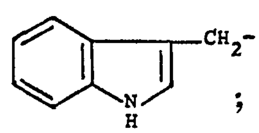 ;

$HOOC—CH_2—CH_2$, 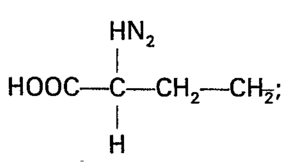 ; oder 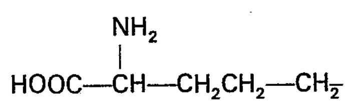

5. Verbindung nach Anspruch 3 oder 4, worin die substituierte Alkylgruppe

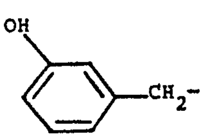 oder 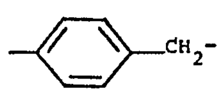

ist.

6. Verbindung nach Anspruch 3, worin die substituierte Alkylgruppe

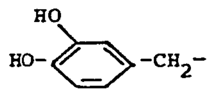 ; 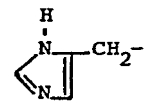 ;

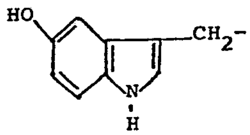 ; 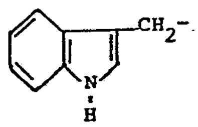 oder $HOOC—CH_2—CH_2—$ ist.

7. Verfahren zur Herstellung von Verbindungen nach Anspruch 1 durch Fluordehydroxylieren einer Verbindung mit der Formel

$$\begin{array}{c} CH_2OH \\ | \\ R—CH—NH_2 \end{array} \qquad VII$$

worin R eine substituierte $C_1—C_4$-Alkylgruppe ist.

8. Verfahren nach Anspruch 7, worin die Verbindung der Formel VII

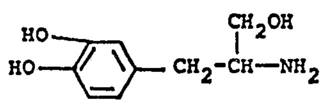 , 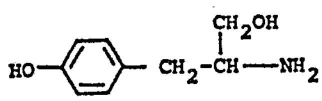 ,

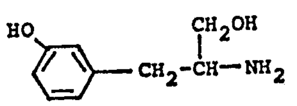 , 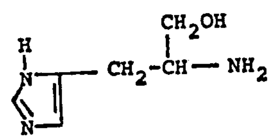 ,

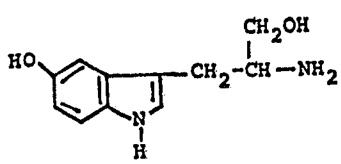 , 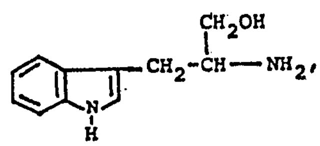 ,

oder 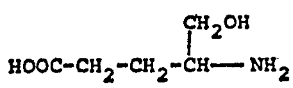 ist.